# EUROPÄISCHE PATENTANMELDUNG

(11) **EP 0 893 164 A2**
(43) Veröffentlichungstag der Anmeldung: **27.01.1999**
(21) Anmeldenummer: 98108662.2
(22) Anmeldetag: 13.05.1998
(51) Int. Cl.: B05D 1/00, A61L 33/00, A61L 27/00

(54) **Bioaktive Beschichtung von Oberflächen unter Mitverwendung von Vernetzern**

(30) Priorität: 28.06.1997 DE 19727556; 04.03.1998 DE 19809054
(71) Anmelder: HÜLS AKTIENGESELLSCHAFT, 45764 Marl (DE)
(72) Erfinder: Kunz, Roland, 45770 Marl (DE); Anders, Christine, Dr., 45721 Haltern (DE); Ottersbach, Peter, Dr., 51570 Windeck (DE)

(57) **Zusammenfassung**

Die Erfindung betrifft ein Verfahren zur Beschichtung von Polymersubstraten, bei dem man
(A) mindestens ein Vinylmonomer der allgemeinen Formel R-(A)ₐ (Monomer A), wobei R einen ein- oder zweifach aliphatisch ungesättigten organischen Rest mit der Wertigkeit a bedeutet; A bestimmte saure Gruppen oder ein Salz davon bezeichnet; a für 1, 2 oder 3 steht und das Monomer A nicht nur eine Carboxylgruppe -COOH oder einer Carboxylatgruppe aufweisen darf, und
(B) mindestens ein mindestens zwei olefinische Doppelbindungen enthaltendes, vernetzendes Vinylmonomer (Monomer B) oder
(C) ein Polymer, das mindestens ein Monomer A enthält (Polymer C), mit mindestens einem Monomer B auf das Polymersubstrat pfropft;
   wobei entweder das Polymersubstrat vor der Pfropfung aktiviert oder ein Initiator mitverwendet und die Pfropfung durch elektromagnetische Strahlung oder thermisch initiiert wird. Erfindungsgemäß beschichtete Erzeugnisse eignen sich zur Verwendung auf den Gebieten der Nahrungs- und Genußmitteltechnik, Wassertechnik, Biotechnik, der Hygienevorsorge und insbesondere der Medizintechnik.

## Beschreibung

Die Erfindung betrifft ein Verfahren zur bioaktiven Beschichtung der Oberfläche von Polymersubstraten durch Pfropfung mit bestimmten Monomeren oder einem Polymer daraus. Wichtige Eigenschaften der erfindungsgemäß aufgebrachten Beschichtungen sind ihre bakterienabweisenden Eigenschaften sowie gute Verträglichkeit im Kontakt mit Körperflüssigkeiten und Gewebe. Je nach Funktionalität der Beschichtungsmonomeren bzw. nach dem Molverhältnis bestimmter funktioneller Gruppen in der Beschichtung werden die Oberflächen darüber hinaus zellproliferationshemmend oder zellproliferationsfördernd eingestellt. Die Erfindung betrifft weiterhin Erzeugnisse mit derart beschichteten Oberflächen zur Verwendung auf den Gebieten der Nahrungs- und Genußmitteltechnik, Wassertechnik, Biotechnik der Hygienevorsorge und insbesondere der Medizintechnik.

### I. Ältere Anmeldungen auf dem Gebiet der bioaktiven Polymeren

1. Gegenstand der deutschen Patentanmeldung 197 23 132.2 (O.Z. 5202) ist die Verwendung von wasserunlöslichen Polymeren A, erhältlich durch radikalische Polymerisation von
   (a) mindestens einem Monomer der allgemeinen Formel 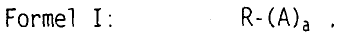 in der
      - R: einen aliphatisch ungesättigten organischen Rest mit der Wertigkeit a bedeutet.
      - A: eine Carboxylgruppe -COOH, Schwefelsäuregruppe -OSO₂OH, Sulfonsäuregruppe -SO₃H, Phosphorsäuregruppe -OPO(OH)₂, Phosphonsäuregruppe -PO(OH)₂, Phosphorigsäuregruppe -OP(OH)₂, phenolische Hydroxylgruppe oder ein Salz einer der genannten Gruppen bezeichnet, und
      - a: für 1, 2 oder 3 steht;
      mit der Maßgabe, daß wenn das Monomer der Formel I eine Carboxylgruppe -COOH oder einer Carboxylatgruppe aufweist, entweder dieses Monomer mindestens einen weiteren Rest A mit einer anderen der für A genannten Bedeutungen enthält oder mindestens ein weiteres Monomer der Formel I. in dem A eine andere der für A genannten Bedeutungen hat, mitverwendet wird; und
   (b) mindestens einem anderen aliphatisch ungesättigten Monomer,
      als bakteriophobes Material.
      Ein weiterer Gegenstand der deutschen Patentanmeldung 197 23 132.2 (O.Z. 5202) sind neue, zu den Polymeren A zählende wasserunlösliche, bakteriophobe Polymere B, erhältlich durch radikalische Copolymerisation von
   (c) einem oder mehreren Carboxylgruppen- und/oder Carboxylatgruppen-haltigen, aliphatisch ungesättigten Monomeren oder den entsprechend funktionalisierten Derivaten der Monomeren als Komponente I mit
   (d) einem oder mehreren Sulfonsäuregruppen- und/oder Sulfonatgruppen-haltigen, aliphatisch ungesättigten Monomeren oder den entsprechend funktionalisierten Derivaten der Monomeren als Komponente II und
   (b) einer Komponente III, die ein weiteres aliphatisch ungesättigtes Monomer oder mehrere weitere aliphatisch ungesättigte Monomere enthält,
      wobei gegebenenfalls die entsprechend funktionalisierten Derivate nach der Copolymerisation zumindest an der Oberfläche in Carboxyl- oder Carboxylatgruppen bzw. Sulfonsäure- oder Sulfonatgruppen überführt werden.

   Des weiteren ist Gegenstand der vorliegenden Erfindung ein Verfahren zur Herstellung der wasserunlöslichen, bakteriophoben Polymeren B.
   Zu den weiteren Monomeren, die als Monomer (b) bzw. als Komponente III mitverwendet werden können, zählen auch Diolefine.
2. Gegenstand der deutschen Patentanmeldung 197 23 131.4 (O.Z. 5203) ist die Verwendung derselben Polymeren A und sind dieselben Polymere B, wobei es jedoch auf die zellproliferationsinhibierende Wirkung ankommt, die bei bestimmten Molverhältnissen von Carboxyl- und/oder Carboxylatgruppen zu Sulfonsäure- und/oder Sulfonatgruppen besonders ausgeprägt ist.
3. Die deutsche Patentanmeldung 197 00 078.9 (O.Z. 5141) betrifft die Polymere B der beiden zuvor genannten Anmeldungen, wobei jedoch auf eine die Zellproliferation begünstigende Wirkung abgehoben wird, die bei bestimmten Molverhältnissen von Carboxyl und/oder Carboxylatgruppen zu Sulfonsäure- und/oder Sulfonatgruppen gegeben ist.
4. Die deutsche Patentanmeldung 197 20 370.1 (O.Z. 5192) betrifft ein Verfahren zur bioaktiven Beschichtung der Oberfläche von Substraten, insbesondere von polymeren Substraten, bei dem man mindestens ein Monomer der allgemeinen Formel 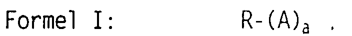 in der R, A und a die unter 1, genannte Bedeutung haben, strahleninduziert auf einer aktivierten Substratoberfläche pfropfpolymerisiert, mit der Maßgabe, daß ein Monomer I, in dem A die Carboxylgruppe -COOH oder ein Salz oder einen Ester der Carboxylgruppe bedeutet, entweder mindestens einen weiteren Rest A mit einer anderen der für A genannten Bedeutungen enthält oder zusammen mit mindestens einem weiteren Monomeren I verwendet wird, in dem A eine andere der für A genannten Bedeutungen hat.
   Dabei setzt man als Monomer der Formel I vorteilhaft eine Mischung von Monomeren der allgemeinen Formeln II oder III 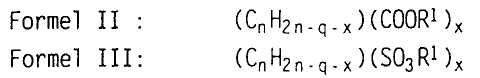 ein. In den Formeln, die in der allgemeinen Formel I eingeschlossen sind, steht
   - n: jeweils unabhängig für eine ganze Zahl von 2 bis einschließlich 6;
   - x: jeweils unabhängig für 1 oder 2;
   - q: jeweils unabhängig für 0 oder 2; und
   bedeutet der Rest R¹ jeweils unabhängig -H, ein Äquivalent eines Metallions, insbesondere ein Alkalimetallion, einen Rest eines aliphatischen, cycloaliphatischen oder araliphatischen Alkohols, vorzugsweise eines Alkanols mit 1 bis 6, insbesondere mit 1 bis 4 Kohlenstoffatomen, eines Cycloalkanols mit 5 bis 12 Kohlenstoffatomen, eines Arylalkanols mit 7 bis 10 Kohlenstoffatomen oder eines Alkanols mit Sauerstoff- und/oder Stickstoffatomen in der Kette und bis zu 12 Kohlenstoffatomen, wie -(CH₂-CH₂-O)_{d}-H, -(CH₂-CH(CH₃)-O)_{d}-H, -(CH₂-CH₂-CH₂-O)_{d}-H oder -(CH₂)_{d}-NH₂₋ₑ(R²)ₑ, wobei R² für -CH₃ oder -C₂H₅, d für 1, 2, 3 oder 4 und e für 0, 1 oder 2 steht.

   Für das Verfahren hat sich eine Kombination aus Monomeren I und II bewährt, die zu Beschichtungen führt, welche einerseits Carboxyl- und/oder Carboxylatgruppen und andererseits Sulfonsäure- und/oder Sulfonatgruppen aufweisen. Diese Beschichtungen zeigen eine gute Blutverträglichkeit und ebenso eine gute bakterienabweisende Wirkung. Sie haben zudem ausgeprägte zellproliferationsinhibierende Eigenschaften, wenn das Verhältnis der genannten Gruppen 0,2 bis 3, vorteilhaft 0,4 bis 3 und insbesondere 0,4 bis 2 beträgt. Die beschichteten Oberflächen zeigen dagegen zellproliferationsfördernde Eigenschaften, wenn das besagte molare Verhältnis 2 bis 10, vorteilhaft 3 bis 10 und insbesondere 3 bis 5 beträgt.
   Auch hier können neben den Monomeren I, II und III mit den wirkungsbestimmenden Gruppen weitere Monomere mitverwendet werden, von denen auch Diolefine genannt werden.
5. Gegenstand der deutschen Patentanmeldung 197 00 081.9 (O.Z. 5143) ist die Herstellung von bakterienabweisenden, kovalent fixierte Beschichtungen auf der Oberfläche von Substraten, insbesondere von Polymersubstraten, wobei man ein Beschichtungspolymer, das
   (i) mindestens ein Monomer der allgemeinen Formel 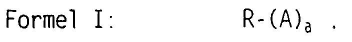 in der R, A und a die zuvor unter 1, genannte Bedeutung haben, und
   (ii) mindestens ein UV-strahlungssensitives Monomer einpolymerisiert enthält,
      strahleninduziert auf eine aktivierte Substratoberfläche pfropft.

   Zu den gepfropften Beschichtungspolymeren zählen wiederum solche, bei denen als Monomere I eine Mischung von Monomeren der allgemeinen Formeln II und III, 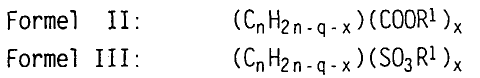 verwendet wird, in denen R¹, n, q und x die zuvor unter 4, genannte Bedeutung haben.
   Neben den Monomeren I, II und III mit den wirkungsbestimmenden Gruppen können wiederum weitere Monomere mitverwendet werden, von denen auch Diolefine genannt werden.
6. Gegenstand der deutschen Patentanmeldung 197.00.82.7 (O.Z. 5144) ist die Herstellung derselben Beschichtungspolymeren wie in der unter 5, genannten Patentanmeldung, wobei es jedoch auf die zellproliferationsinhibierende Wirkung ankommt, die bei bestimmten Molverhältnissen von Carboxyl- und/oder Carboxylatgruppen zu Sulfonsäure- und/oder Sulfonatgruppen besonders ausgeprägt ist.
7. Die deutsche Patentanmeldung 197 00 83.5 (O.Z. 5145) betrifft ein Verfahren zur Herstellung von bakterienabweisenden und zugleich die Zellproliferation begünstigenden, kovalent fixierten Beschichtungen auf der Oberfläche von Substraten, bei dem man ein Beschichtungspolymer, das
   (i) mindestens ein Monomer der allgemeinen Formel I 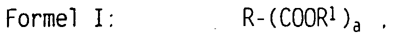 in der
      - R: einen ein- oder zweifach olefinisch ungesättigten organischen Rest mit der Wertigkeit a bedeutet,
      - R¹: Wasserstoff, ein Metalläquivalent oder einen organischen Rest bedeutet und
      - a: für 1, 2 oder 3 steht;
   (ii) mindestens ein Monomer der allgemeinen Formel II 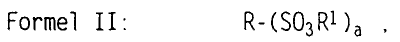 in der R, R¹ und a die angegebene Bedeutung haben; und
   (iii) mindestens ein Monomer mit einer UV-strahlungssensitiven Gruppe
      einpolymerisiert enthält, strahleninduziert auf eine aktivierte Substratoberfläche aufpfropft, wobei das molare Verhältnis der Reste -COOR¹ und -SO₃R¹ 3 bis 10 beträgt.

   Auch bei diesem Verfahren können zusätzlich zu den Monomeren I und II weitere Monomere ohne wirkungsbestimmende Gruppen mitverwendet werden, darunter wiederum Diolefine.
8. Schließlich betrifft die deutsche Patentanmeldung 197 15 449.2 (O.Z. 5176) ein Verfahren zur Modifizierung der Oberfläche von Polymersubstraten mittels photochemisch induzierter Pfropfpolymerisation, bei dem man das Polymersubstrat zunächst mit einem Photoinitiator und mindestens einem aliphatisch ungesättigten Monomeren vorbehandelt und dann auf das vorbehandelte Polymersubstrat durch elektromagnetische Strahlung induziert mindestens ein aliphatisch ungesättigtes Monomer polymerisiert wird. Zu den verwendbaren aliphatisch ungesättigten Monomeren zählen auch solche, die den zuvor erwähnten Formeln I, II und III entsprechen. Neben monoolefinischen Monomeren können auch diolefinische Monomere der Formel CH₂=CR¹-R⁶-CR¹=CH₂, in der R¹ für Wasserstoff oder eine Methylgruppe steht und R⁶ einen zweiwertigen organischen Rest bedeutet, einpolymerisiert werden, wodurch vernetzte Pfropfcopolymere entstehen.

### II. Beschreibung der Erfindung

Es wurde nun überraschenderweise gefunden, daß sich Polymersubstrate durch Pfropfung mit Monomeren oder Polymeren mit einer gegenüber den Verfahren der älteren Patentanmeldungen stärker bakterienabweisenden und besser haftenden Beschichtung versehen lassen, wenn man
(A) mindestens ein Vinylmonomer (Monomer A) der allgemeinen Formel 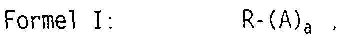 in der
   - R: einen ein- oder zweifach olefinisch ungesätttigten organischen Rest mit der Wertigkeit a bedeutet, A eine Carboxylgruppe -COOH, Schwefelsäuregruppe -OSO₂OH, Sulfonsäuregruppe -SO₃H, Phosphorsäuregruppe -OPO(OH)₂, Phosphonsäuregruppe -PO(OH)₂, Phosphorigsäuregruppe -OP(OH)₂, phenolische Hydroxylgruppe oder ein Salz einer der genannten Gruppen bezeichnet, und
   - a: für 1, 2 oder 3 steht;
   mit der Maßgabe, daß wenn das Monomer der Formel I eine Carboxylgruppe -COOH oder einer Carboxylatgruppe aufweist, entweder dieses Monomer mindestens einen weiteren Rest A mit einer anderen der für A genannten Bedeutungen enthält oder mindestens ein weiteres Monomer der Formel I, in dem A eine andere der für A genannten Bedeutungen hat, mitverwendet wird; und
(B) mindestens ein mindestens zwei olefinische Doppelbindungen enthaltendes, vernetzendes Vinylmonomer (Monomer B) oder
(C) ein Polymer, das mindestens ein Monomer A enthält (Polymer C), mit mindestens einem Monomer B auf das Polymersubstrat pfropft; wobei entweder das Polymersubstrat vor der Pfropfung aktiviert wird und die Pfropfung durch elektromagnetische Strahlung oder thermisch initiiert wird oder
   ein Initiator mitverwendet und die Pfropfung durch elektromagnetische Strahlung oder thermisch initiiert wird.

Man kann also wahlweise ein vorgefertigtes, unvernetztes Polymer zusammen mit einem Vernetzer pfropfen oder aber von den Monomeren ausgehen und so verfahrenstechnisch vorteilhaft die Polymerisation mit der Pfropfung verbinden.

Unter den Salzen der für A in der Formel I genannten Gruppen werden Alkalisalze und insbesondere die Natriumsalze bevorzugt.

Das gemeinsame Kennzeichen der Monomeren der Formel I (Monomere A) ist, daß sie 1 oder 2 olefinische Doppelbindungen sowie mindestens eine saure Gruppe oder ein Salz einer sauren Gruppe aufweisen.

Die erfindungsgemäß beschichteten Oberflächen zeigen eine bemerkenswerte Kombination vorteilhafter Eigenschaften und daher eine hervorragende physiologische Verträglichkeit. Sie sind insbesondere besser blutverträglich und vermindern die Adhäsion und Vermehrung von Bakterien in einem höheren Maße auch über längere Zeit, als dies bei den Beschichtungen nach den erwähnten Patentanmeldungen der Fall ist. Dies gilt u.a. für die Bakterien Staphylococcus aureus, Staphylococcus epidermidis, Streptococcus pyogenes, Klebsiella pneumoniae, Pseudomonas aeruginosa und Escherichia coli. Gleichzeitig wird meistens auch die Proliferation von Zellen inhibiert, beispielsweise von Nabelschnurzellen. Die besonderen Bedingungen, unter denen eine Beschichtung bakterienabweisend, aber zellproliferationsfördernd ist, werden später erläutert. Die Oberflächen der erfindungsgemäß beschichteten Substrate sind, gegebenenfalls nach Extraktion, frei von migrationsfähigen und/oder extrahierbaren Monomer- und Oligomeranteilen. Unerwünschte Nebenwirkungen durch freigesetzte körperfremde Stoffe oder durch abgetöte Bakterien werden nicht beobachtet.

### 1. Monomere A

Bei der Pfropf(co)polymerisation von Monomeren setzt man z.B. als Monomer A vorteilhaft eine Mischung von Monomeren der allgemeinen Formeln II oder III 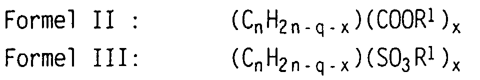 ein. In den Formeln, die in der allgemeinen Formel I eingeschlossen sind, steht
- n: jeweils unabhängig für eine ganze Zahl von 2 bis einschließlich 6;
- x: jeweils unabhängig für 1 oder 2;
- q: jeweils unabhängig für 0 oder 2; und
bedeutet der Rest R¹ jeweils unabhängig -H oder ein Äquivalent eines Metallions, insbesondere ein Alkalimetallion.

Den gegebenen Definitionen entsprechend bedeutet der Rest (CₙH_{2n-q-x})- jeweils unabhängig einen geradkettigen oder verzweigten einwertigen Alkenylrest (q=0, x=1) oder Alkadienylrest (q=2, x=1) oder einen zweiwertigen Alkenylenrest (q=0, x=2) oder Alkadienylenrest (q=2, x=2).

Anstelle von zwei Monomeren A mit den Formeln II und III kann man auch nur ein Monomer (II + III) einsetzen, das die Gruppen -COOR¹ und -SO₃R¹ in demselben Molekül enthält.

Auch vom Benzol abgeleitete Monomere A der allgemeinen Formel 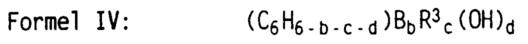 können eingesetzt werden, worin
- B: jeweils unabhängig einen ein- oder zweifach ungesättigten geradkettigen oder verzweigten Rest der Formeln (CₙH_{2n-1-q-y})(COOR¹)_{y} oder (CₙH_{2n-1-q-y})(SO₃R¹)_{y} bedeutet, wobei R¹, n und q wie zuvor definiert sind und y für 0, 1 oder 2 steht;
- R³: jeweils unabhängig C₁₋₄-Alkyl, -NH₂, -COOH, -SO₃H, -OSO₃H, -OPO(OH)₂, -PO(OH)₂, -OP(OH)₂, -OPO(O⁻)OCH₂-CH₂-N⁺(CH₃)₃, -PO(O⁻)O-CH₂-CH₂-N⁺(CH₃)₃, -OP(O⁻)OCH₂-CH₂-N⁺(CH₃)₃ oder ein Salz, insbesondere ein Alkalisalz, oder einen Ester der genannten Gruppen bedeutet;
- b: für 1, 2, oder 3 steht;
- c: für 0, 1, 2, oder 3 steht; und
- d: für 0, 1, 2, oder 3 steht;
mit der Maßgabe, daß b + c + d ≤ 6, vorteilhaft ≤4 ist.

Natürlich können außer der erwähnten Kombination von Monomeren A der Formeln II und III auch beliebige Mischungen von Monomeren A der allgemeinen Formeln I, II, III und IV für das erfindungsgemäße Verfahren eingesetzt werden.

Andere geeignete Monomere A sind der Formel I entsprechende Verbindungen mit Schwefelsäuregruppen oder Salzen dieser Gruppen (Sulfatgruppen); Sulfonsäuregruppen oder deren Salzen; Phosphonsäuregruppen, sauren Phosphonsäureestergruppen oder Salzen von Phosphonsäuregruppen oder sauren Phosphonsäureestergruppen; Phosphorsäuregruppen, sauren Phosphorsäureestergruppen oder Salzen von Phosphorsäuregruppen oder sauren Phosphorsäureestergruppen; Phosphorigsäuregruppen, sauren Phosphorigsäureestergruppen oder Salzen von Phosphorigsäuregruppen oder sauren Phosphorigsäureestergruppen. Auch diese Monomeren können im Gemisch untereinander und/oder mit anderen Monomeren A der allgemeinen Formeln I, II, III und IV für das erfindungsgemäße Verfahren verwendet werden.

Weiterhin seien noch 1 bis 3-wertige (oder -basische) Phenole sowie deren Salze, die der Formel I entsprechen, als geeignete Monomere erwähnt. Auch sie werden gegebenenfalls im Gemisch untereinander und/oder mit den zuvor erwähnten Monomeren A eingesetzt.

Für das erfindungsgemäße Verfahren hat sich eine Kombination aus Monomeren I bis IV bewährt, die zu Beschichtungen führt, welche einerseits Carboxyl- und/oder Carboxylatgruppen und andererseits Sulfonsäure- und/oder Sulfonatgruppen aufweisen. Es gibt unter dem Aspekt der Verträglichkeit bzw. Koexistenz drei mögliche Zweierkombinationen aus den genannten Gruppen, nämlich Carboxyl- und Sulfonsäuregruppen, Carboxyl- und Sulfonatgruppen sowie Carboxylat- und Sulfonatgruppen, und weiterhin zwei Dreierkombinationen, nämlich Carboxyl-, Carboxylat- und Sulfonatgruppen sowie Carboxyl-, Sulfosäure- und Sulfonatgruppen. Alle diese Kombinationen charakterisieren brauchbare Ausführungsformen des erfindungsgemäßen Verfahrens. Natürlich ist es auch möglich. Monomere einzusetzen, deren funktionelle Gruppen nach der Pfropfpolymerisation verändert werden. So kann man z.B. den Acrylamid-Baustein nachträglich durch Hydrolyse in saurem Medium in den Acrylsäure-Baustein umwandeln. Weiterhin kann man Carboxyl- und Sulfonsäuregruppen durch Neutralisieren (z.B. in Phosphatpuffern) sowie Carbonester- und Sulfosäureestergruppen durch Hydrolyse in Carboxylat- bzw. Sulfonatgruppen überführen. In der genannten Kombination kann das molare Verhältnis von Carboxyl- und/oder Carboxylatgruppen zu Sulfonsäure- und/oder Sulfonatgruppen in der Beschichtung in weiten Grenzen schwanken. Besonders ausgeprägte zellproliferationsinhibierende Eigenschaften werden erzielt, wenn das genannte Verhältnis 0,2 bis 3, vorteilhaft 0,4 bis 3 und insbesondere 0,4 bis 2 beträgt. Die beschichteten Oberflächen zeigen in bemerkenswerter Weise bakterienabweisende, aber zellproliferationsfördernde Eigenschaften, wenn das besagte molare Verhältnis 2 bis 10, vorteilhaft 3 bis 10 und insbesondere 3 bis 5 beträgt. Zellproliferationsfördernd im Sinne der Erfindung ist eine Beschichtung dann, wenn die Haftung und Vermehrung von Säugetierzellen durch die Beschichtung im Vergleich zu der unbeschichteten Oberfläche verbessert oder jedenfalls weniger stark beeinträchtigt wird als die Haftung und Vermehrung von Bakterien.

Von den geeigneten Monomeren A seien beispielsweise genannt: Acrylsäure, 4-Vinylsalicylsäure, Itaconsäure, Vinylessigsäure, Zimtsäure, 4-Vinylbenzoesäure, 2-Vinylbenzoesäure, Sorbinsäure, Kaffeesäure, Maleinsäure, Methylmaleinsäure, Crotonsäure, Isocrotonsäure, Fumarsäure, Dimethylfumarsäure, Methylfumarsäure, Dihydroxymaleinsäure, Allylessigsäure sowie die Natriumsalze dieser Säuren; Natriumallylsulfat, Natriumallylsulfonat, Natriummethallylsulfat säure, Natriumstyrolsulfonat, Natriumvinyltoluolsulfonat; Buten-(2)-diol-(1,4)-diphosphorsäure, Buten-(2)-diol-(1,4)-diphosphonsäure sowie die Dinatriumsalze der beiden Phosphor- bzw. Phosphonsäuren, Monoallylphosphit; 2-Vinylphenol, 2-Allylhydrochinon, 4-Vinylresorcin, m-Hydroxystyrol, o-Hydroxystyrol, p-Hydroxystyrol, Carboxyl-vinylbenzolsulfonsäure.

Bei einer weiteren Ausführungsform des erfindungsgemäßen Verfahrens verwendet man als Monomer A eine Mischung aus Monomeren der allgemeinen Formeln V und VI

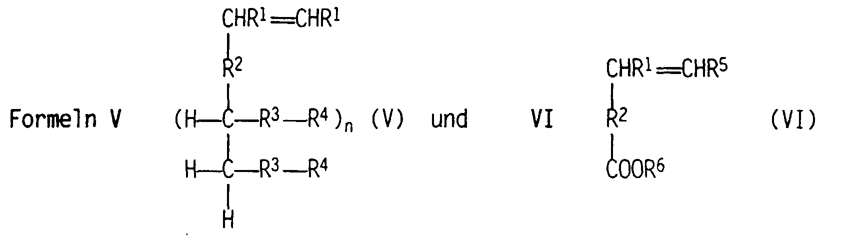

In den Formeln I und II bedeuten
- R¹: Wasserstoff oder den Methylrest,
- R²: einen zweiwertigen organischen Rest, vorzugsweise einen aliphatischen, cycloaliphatischen oder aromatischen Kohlenwasserstoffrest mit bis zu 10 Kohlenstoffatomen, oder eine C-C-Einfachbindung,
- R³: -O- oder -NH-,
- R⁴: Wasserstoff oder den Rest -SO₃⁻Na⁺,
- R⁵: Wasserstoff, den Methylrest oder den Rest -R²-COO-Na⁺,
- R⁶: Wasserstoff oder Na und
- n: 4 oder 5;
mit der Maßgabe, daß mindestens einer der Substituenten R⁴ ein Rest -SO₃⁻Na⁺ ist.

In bevorzugten Monomeren A der Formeln V und VI bedeutet
- R¹: Wasserstoff,
- R²: einen Alkylenrest mit 1 bis 4 Kohlenstoffatomen, einen Phenylenrest oder eine C-C-Einfachbindung,
- R³: -O- oder -NH-,
- R⁴: Wasserstoff oder den Rest -SO₃⁻Na⁺,
- R⁵: Wasserstoff oder den Rest -R²-COO⁻Na⁺,
- R⁶: Wasserstoff oder Na und steht
- n: für 4.

Die Monomeren A der Formel V enthalten modifizierte Zuckerreste, die vorzugsweise von Pentosen und insbesondere von Arabinose abgeleitet sind. Die Zuckerreste enthalten mindestens einen der Reste -O-SO₃⁻Na⁺ ("O-Sulfat") oder -NH-SO₃⁻Na⁺ ("N-Sulfat"), bevorzugt benachbart zu dem Rest R². Sie weisen vorzugsweise 1 bis 4 dieser Reste auf. In einem Zuckerrest können O-Sulfat- und N-Sulfatreste gleichzeitig vorliegen, wobei dann der N-Sulfatrest bevorzugt benachbart zum Rest R² positioniert ist. Alternativ kann der Zuckerrest aber auch ausschließlich eine Art dieser Reste enthalten, z.B. nur O-Sulfatreste. Jede der genannten Spezies (nur O-Sulfat enthaltende sowie N-Sulfat-haltige Reste) ist für sich allein oder zusammen mit der anderen Spezies als Monomer A der Formel V geeignet. Das Mischungsverhältnis beträgt also 0:100 bis 100:0.

### 2. Vernetzende Vinylmonomere B

Vernetzende Vinylmonomere (Monomere B) weisen mindestens 2, vorteilhaft 3 bis 6 olefinische Doppelbindungen auf und haben im allgemeinen keine Gruppen, die zu der biologischen Wirkung der Beschichtung beitragen. Ihre Mitverwendung führt zu dickeren und delaminationsstabileren Schichten und setzt die Bakterienadhäsion und -vermehrung unter sonst gleichen Bedingungen noch einmal erheblich herab. Vernetzer mit mehr als 2, insbesondere solche mit 3 bis 6 olefinischen Doppelbindungen, sind insoweit deutlich wirksamer als diolefinische Vernetzer. Ihre Mitverwendung ist auch dann nützlich, wenn die Monomeren A mehr als eine olefinische Doppelbindung enthalten, also allein ein Netzwerk bilden könnten. Natürlich kann man auch mit zwei oder mehr verschiedenen Monomeren B arbeiten. Die Monomeren B können neben ihren olefinischen Doppelbindungen z.B. hydrophile Gruppen, wie Hydroxylgruppen und/oder Alkylenoxidgruppen, enthalten und sind dann gleichzeitig hydrophilierende und vernetzende Monomere B. Man wendet die Monomeren B zweckmäßig in Mengen von 0.01 bis 50 Molprozent, vorteilhaft von 0,1 bis 20 Molprozent an, bezogen auf die Monomeren A.

Geeignete Monomere B sind z.B. solche mit 2 olefinischen Doppelbindungen, wie 1,3-Butadien, Isopren und Chloropren; (Meth)crylsäureester, wie Ethylenglykoldiacrylat, Diethylenglykoldimethacrylat und Polyethylenglykol-1000-dimethacrylat; und Vinylverbindungen, wie 1,4-Butandioldivinylether, Ethylenglykoldivinylether und 1,4-Divinylbenzol. Von den Vernetzern mit 3 oder mehr olefinischen Doppelbindungen seien beispielsweise Acrylsäureester, wie Trimethylolpropantriacrylat, Trimethylolpropantrimethacrylat und Pentaerythrittetraacrylat; Polyvinylether, wie Glycerin-12EO-trivinylether und Pentaerythrit-64EO-tetravinylether; Kohlenhydratderivate, wie acryloylierte Hydroxypropylzellulose mit mehr als zwei Acryloylgruppe pro Molekül; und Allylverbindungen, wie Pentaerythrittetraallylether genannt.

### 3. Weitere, gegebenenfalls mitverwendbare Monomere D

Neben den beschriebenen Monomeren A mit den aufgeführten blutverträglich machenden und bakterienabweisend sowie zellproliferationsinhibierend oder -fördernd wirkenden Gruppen und den Vernetzern B kann man auch andere, einfach olefinisch ungesättigte Monomere (in der Folge Monomere D genannt) mitverwenden, die insoweit neutral oder allenfalls schwach wirksam sind. Das können z.B. (Meth)acrylsäurederivate sein, wie Ethylacrylat, Isobutylacrylat, 2-Ethylhexylacrylat, 2-Hydroxyethylacrylat, 2-(2'-Hydroxyethoxy)ethylacrylat, 2-Hydroxy-1-methylethylacrylat, 2-N,N-Dimethylaminoethylacrylat, n-Propylmethacrylat, 2-Hydroxyethylmethacrylat, 2-(2'-Hydroxyethoxy)ethylmethacrylat, 2-Hydroxy-1-methylethylmethacrylat, 2-N,N-Dimethylaminoethylmethacrylat, Diethylenglykolmonomethacrylat, (Meth)acrylnitril; Vinylketone, wie Vinylethylketon; Olefine und Diolefine, wie 1-Buten und 1-Hexen; Vinylaromaten, wie Styrol, Vinyltoluol und Divinylbenzol; und Vinylsiloxane. Diese Monomeren können sogar in überwiegender Menge vorhanden sein, z.B. bis zu 90 Mol-% ausmachen.

### 4. Polymere C aus den Monomeren A und gegebenenfalls D

Statt die Monomeren A, B und gegebenenfalls D als solche zu pfropfen, kann man auch die Monomeren A und gegebenenfalls D vorher polymerisieren und die so erhaltenen Polymere (Polymere C) zusammen mit einem vernetzenden Monomer B pfropfen (oder pfropfpolymerisieren). Die Polymeren C werden in üblicher Weise durch radikalisch initiierte Polymerisation hergestellt, vorteilhaft durch Lösungs- oder Emulsionspolymerisation. Geeignete Lösemittel sind z.B. Wasser; Ketone, wie Aceton, Methylethylketon, und Cyclohexanon; Ether, wie Diethylether, Tetrahydrofuran und Dioxan; Alkohole, wie Methanol, Ethanol, n- und iso-Propanol, n- und iso-Butanol und Cyclohexanol; stark polare Lösemittel, wie Dimethylformamid, Dimethylacetamid und Dimethylsulfoxid; Kohlenwasserstoffe, wie Heptan, Cyclohexan, Benzol und Toluol; Halogenkohlenwasserstoffe, wie Dichlormethan und Trichlormethan; Ester, wie Ethylacetat, Propylacetat und Amylacetat; sowie Nitrile, wie Acetonitril; oder Gemische aus diesen Lösemitteln.

Geeignete Polymerisationsinitiatoren sind z.B. Azonitrile, Alkylperoxide, Acylperoxide, Hydroperoxide, Peroxyketone, Peroxyester und Percarbonate sowie alle üblichen Photoinitiatoren. Die Polymerisation wird thermisch, z.B. durch Erhitzen auf 60 bis 100°C, oder durch Strahlung mit entsprechender Wellenlänge eingeleitet. Nach Beendigung der exothermen Polymerisationsreaktion wird das Polymer C in üblicher Weise vom Lösemittel abgetrennt, beispielsweise durch Fällung mittels Ethanol, sofern die Polymerisation wäßriger Lösung durchgeführt wurde. In Emulsion hergestellte Polymere können durch Sprühtrocknung gewonnen werden. Durch Extraktion mit einem geeigneten Lösemittel können monomere oder oligomere Bestandteile entfernt werden.

### 5. Die Polymersubstrate

Als Polymersubstrate eignen sich alle polymeren Kunststoffe, wie Polyurethane, Polyamide, Polyester und -ether, Polyetherblockamide und Polyorganosiloxane. Weiterhin geeignet sind z.B. Polystyrol, Polyvinylchlorid, Polycarbonate, Polyolefine, Polysulfone, Polyisopren, Polychloropren, Polytetrafluorethylen (PTFE), Polyacrylate, Polymethacrylate, entsprechende Copolymere und Blends sowie natürliche und synthetische Kautschuke, mit oder ohne strahlungssensitive Gruppen. Das erfindungsgemäße Verfahren läßt sich auch auf Oberflächen von lackierten oder anderweitig mit Kunststoff beschichteten Metall-, Glas- oder Holzkörpern anwenden.

### 6. Pfropfung auf die Polymersubstrate

### 6.1 Auf aktivierte Substratoberflächen

Die Oberflächen der Substrate können erfindungsgemäß nach einer Reihe von Methoden aktiviert werden. Zweckmäßig werden sie zuvor in bekannter Weise mittels eines Lösemittels von Ölen, Fetten oder anderen Verunreinigungen befreit.
6.1.1 Die Aktivierung von Standardpolymeren ohne UV-strahlungssensitive Gruppen kann vorteilhaft durch UV-Strahlung. z.B. im Wellenlängenbereich von 100 bis 400 nm, vorzugsweise von 125 bis 310 nm erfolgen. Eine geeignete Strahlenquelle ist z.B. ein UV-Excimer-Gerät HERAEUS Noblelight, Hanau, Deutschland. Aber auch Quecksilberdampflampen eignen sich zur Substrataktivierung, sofern sie erhebliche Strahlungsanteile in den genannten Bereichen emittieren. Die Expositionszeit beträgt im allgemeinen 0,1 Sekunden bis 20 Minuten, vorzugsweise 1 Sekunde bis 10 Minuten. Es hat sich gezeigt, daß die Anwesenheit von Sauerstoff vorteilhaft ist. Die bevorzugten Sauerstoffdrücke liegen zwischen 2x10⁻⁵ und 2x10⁻² bar. Man arbeitet beispielsweise in einem Vakuum von 10⁻⁴ bis 10⁻¹ bar oder unter Verwendung eines Inertgases, wie Helium, Stickstoff oder Argon, mit einem Sauerstoffgehalt von 0,02 bis 20 Promille.
6.1.2 Die Aktivierung kann erfindungsgemäß auch durch ein Hochfrequenz- oder Mikrowellenplasma (Hexagon, Fa. Technics Plasma, 85551 Kirchheim, Deutschland) in Luft, Stickstoff- oder Argon-Atmosphäre erreicht werden. Die Expositionszeiten betragen im allgemeinen 30 Sekunden bis 30 Minuten, vorzugsweise 2 bis 10 Minuten. Der Energieeintrag liegt bei Laborgeräten in der Regel zwischen 100 und 2.000W, vorzugsweise zwischen 200 und 1.800W.
6.1.3 Weiterhin lassen sich auch Korona-Geräte (Fa. SOFTAL, Hamburg, Deutschland) zur Aktivierung verwenden. Die Expositionszeiten betragen in diesem Falle in der Regel 1 Sekunde bis 10 Minuten, vorzugsweise 1 bis 60 Sekunden.
6.1.4 Die Aktivierung durch Elektronen- oder gamma-Strahlen (z.B. aus einer Kobalt-60-Quelle) ermöglicht kurze Expositionszeiten, die im allgemeinen 0,1 bis 60 Sekunden betragen.
6.1.5 Beflammungen von Oberflächen führen ebenfalls zu deren Aktivierung, Geeignete Geräte, insbesondere solche mit einer Barriere-Flammenfront, lassen sich auf einfache Weise bauen oder beispielsweise beziehen von der Fa. ARCOTEC, 71297 Mönsheim, Deutschland. Sie können mit Kohlenwasserstoffen oder Wasserstoff als Brenngas betrieben werden. In jedem Fall muß eine schädliche Überhitzung des Substrats vermieden werden, was durch innigen Kontakt mit einer gekühlten Metallfläche auf der von der Beflammungsseite abgewandten Substratoberfläche leicht erreicht wird. Die Aktivierung durch Beflammung ist dementsprechend auf verhältnismäßig dünne, flächige Substrate beschränkt. Die Expositionszeiten belaufen sich im allgemeinen auf 0,1 Sekunde bis 1 Minute, vorzugsweise 0,5 bis 2 Sekunden, wobei es sich ausnahmslos um nicht leuchtende Flammen handelt und die Abstände der Substratoberflächen zur äußeren Flammenfront 0,2 bis 5 cm, vorzugsweise 0,5 bis 2 cm betragen.
6.1.6 Schließlich lassen sich die Oberflächen der Polymersubstrate auch durch Behandlung mit starken Säuren oder starken Basen aktivieren. Von den geeigneten starken Säuren seien Schwefelsäure, Salpetersäure und Salzsäure genannt. Man kann z.B. Polyamide 5 Sekunden bis 1 Minute mit konzentrierter Schwefelsäure bei Raumtemperatur behandeln. Als starke Basen eignen sich besonders Alkalimetallhydroxide in Wasser oder einem organischen Lösemittel. So kann man z.B. verdünnte Natronlauge 1 bis 60 Minuten bei 20 bis 80°C auf die Substrate einwirken lassen. Alternativ können beispielsweise Polyamide aktiviert werden, indem man 2 %iges KOH in Tetrahydrofuran 1 Minute bis 30 Minuten auf die Oberfläche einwirken läßt.

In manchen Fällen, z.B. bei hochhydrophoben Polymeren, kann es empfehlenswert sein, die Oberflächen der Polymersubstrate durch eine Kombination aus zwei oder mehr der genannten Methoden zu aktivieren. Bevorzugte Aktivierungsmethoden sind die unter 6.1.1 und 6.1.2 genannten.

Nachdem die Polymersubstrate nach einer der unter 6.1.1 bis 6.1.6 beschriebenen Methoden vorbehandelt wurden, können die aktivierten Oberflächen 1 bis 20 Minuten, vorzugsweise 1 bis 5 Minuten der Einwirkung von Sauerstoff, z.B. in Form von Luft, ausgesetzt werden. Anschließend werden auf die aktivierten Oberflächen nach bekannten Methoden, wie Tauchen, Sprühen oder Streichen, Beschichtungslösungen aufgebracht. Das können Lösungen der Monomeren A, B und gegebenenfalls D sein. Alternativ bringt man auf das aktivierte Substrat die Lösung eines Polymers C und eines vernetzenden Monomers B auf. Als Lösemittel haben sich insbesondere bei hydrophilen Monomeren bzw. Polymeren Wasser und Wasser-Ethanol-Gemische bewährt, doch sind auch andere Lösemittel verwendbar, sofern sie ein ausreichendes Lösevermögen für die Monomeren bzw. Polymeren aufweisen und die Substratoberflächen gut benetzen. Je nach Löslichkeit der Monomeren und gewünschter Schichtdicke der fertigen Beschichtung können die Konzentrationen der Monomeren bzw. des Polymers in der Lösung 0,1 bis 50 Gewichtsprozent betragen. Lösungen mit Gehalten an Monomeren bzw. an Polymer von 3 bis 10 Gew.-%, beispielsweise mit etwa 5 Gew.-%, haben sich in der Praxis bewährt.

Die Pfropfung kann durch Bestrahlung des in die Beschichtungslösung getauchten Substrats bewirkt werden. Das Substrat kann aber auch zunächst aus der Beschichtungslösung entfernt (oder auf andere übliche Weise, wie Spritzen, Rakeln, Streichen oder Spin-Coating beschichtet werden) und nach dem Verdampfen des Lösemittels oder auch schon während des Verdampfens bestrahlt werden. Man arbeitet zweckmäßig mit Strahlen im kurzwelligen Segment des sichtbaren Bereiches oder im langwelligen Segment des UV-Bereiches der elektromagnetischen Strahlung. Gut geeignet ist z.B. die Strahlung eines UV-Excimers der Wellenlängen 250 bis 500 nm, vorzugsweise von 290 bis 320 nm. Auch hier sind Quecksilberdampflampen geeignet, sofern sie erhebliche Strahlungsanteile in den genannten Bereichen emittieren. Die Expositionszeiten betragen im allgemeinen 10 Sekunden bis 30 Minuten, vorzugsweise 2 bis 15 Minuten.

Alternativ kann die Pfropfung auch thermisch initiiert werden. Dazu erhitzt man das aktivierte Substrat, nachdem es auf übliche Weise mit der Beschichtungslösung beschichtet wurde, z.B. 5 Minuten bis 4 Stunden auf eine Temperatur, die zwischen 50 und 100°C liegen kann.

Bisweilen ist es zweckmäßig die beschriebenen Arbeitsschritte einschließlich der Aktivierung zu wiederholen, um mittels einer solchen Mehrschichttechnik eine hermetisch geschlossene und/oder noch dickere Beschichtung zu erzeugen. Alternativ ist es auch möglich, das aktivierte Polymersubstrat, gegebenenfalls nach der beschriebenen Sauerstoffbehandlung, in die Lösung der Monomeren A, B und gegebenenfalls D bzw. des entsprechenden Polymers C einzutauchen und im getauchten Zustand zu bestrahlen. Durch orientierende Versuche läßt sich unschwer feststellen, bei welchen Bestrahlungszeiten mit einer gegebenen Strahlenquelle und bei welchen, gegebenenfalls längeren Kontaktzeiten von Substrat und Lösung die gewünschte Schichtdicke erreicht wird.

### 6.2 Pfropfung mittels eines niedermolekularen Initiators

Die bei dieser Beschichtungsvariante verwendeten Initiatoren sind niedermolekulare Verbindungen (also keine Makroinitiatoren) mit einer Gruppe, die unter dem Einfluß von elektromagnetischer Strahlung oder von Wärme unter Radikalbildung zerfällt. Beide Arten von Initiatoren sind in der Polymerisationstechnik gut bekannt.

Von den geeigneten Initiatoren, die beim Erhitzen unter Radikalbildung zerfallen, seien z.B. Azonitrile; Alkylperoxide, Acylperoxide, Hydroperoxide, Peroxodisulfate, Peroxyketone, Peroxyester und Percarbonate genannt.

Geeignete Initiatoren für die durch elektromagnetische Strahlung initiierte Pfropfung sind alle gängigen Photoinitiatoren, wie Benzoine, Benzilketale, α-Hydroxyketone, Peroxide, Azoverbindungen, Azoxyverbindungen, Diazosulfonate, Diazosulfone, Diazothioether, Diacyldiazomethane, Diarylsulfide, heteroaromatisch substituierte Disulfide, Diaroylsulfide, Tetraalkylthiuramdisulfide, Dithiocarbonate oder Dithiocarbamate. Im einzelnen seien Benzophenon, Acetophenon, Fluorenon, Benzaldehyd, Propiophenon, Anthrachinon, Carbazol, 3- oder 4-Methylacetophenon, 4,4'-Dimethoxybenzophenon, Allylacetophenon, 2,2'-Diphenoxyacetophenon, Benzoinmethylether, Benzoinethylether, Benzoinpropylether, Benzoinacetat, Benzoinphenylcarbamat, Benzoinacrylat, Benzoinphenylether, Benzoylperoxid, Dicumylperoxid, Azobisisobutyronitril, Phenyldisulfid, Acylphosphanoxide oder Chlormethylanthrachinon sowie Kombinationen hiervon genannt. Bevorzugte, besonders kurze Bestrahlungszeiten ermöglichende Photoinitiatoren sind Benzoine, Benzoinderivate, Benzilketale und α-Hydroxyketone.

Vor der Pfropfung wird das Polymersubstrat mit dem Initiator vorbehandelt. Das kann geschehen, indem man eine Lösung des Initiators auf das Polymersubstrat aufbringt und das Lösemittel verdampft. Geeignete Lösemittel sind, je nach Substrat und Initiator, z.B. Wasser, Aceton, Methylethylketon, Butanon, Cyclohexanon, Diethylether, Tetrahydrofuran, Dioxan, Methanol, Ethanol, Propanol, Butanol, Cyclohexanol, Dimethylacetamid, Dimethylsulfoxid, Dimethylformamid, Heptan, Cyclohexan, Benzol, Toluol, Dichlormethan, Trichlormethan, Ethylacetat, Propylacetat, Amylacetat oder Acetonitril. Das Lösemittel soll das Polymersubstrat anquellen, aber nicht lösen, so daß ein inniger Kontakt von Polymersubstrat und Initiator geschaffen wird.

Im Interesse einer guten Haftung der biologisch aktiven Beschichtung auf dem Polymersubstrat ist es jedoch zweckmäßig, das Polymersubstrat nach der Lehre der deutschen Patentanmeldung 197 15 449.2 (O.Z. 5176) zunächst mit einem Initiator und mindestens einem Monomer vorzubehandeln und dann die Monomeren A, B und gegebenenfalls D oder alternativ ein Polymer C und ein Monomer B auf das vorbehandelte Polymersubstrat zu pfropfen. Die Wahl des Initiators und des Monomers richtet sich u.a. nach der chemischen Natur des Polymersubstrats. Das Monomer muß das Polymersubstrat anquellen können und damit die Penetration des Initiators in die oberflächennahen Zonen des Polymersubstrats ermöglichen. Ob das Monomer für die Vorbehandlung die erwünschten biologischen Eigenschaften vermittelt, spielt dagegen keine Rolle. Man kann also durchaus mit einem Monomer vorbehandeln, das das Polymersubstrat gut anquillt und den Initiator gut löst und penetrieren läßt, aber nicht die letztlich gewünschten biologischen Eigenschaften ergibt, und erst in der Pfropfphase die erwähnten Monomeren mit den biologisch wirksamen Gruppen bzw. ein Polymer daraus einzusetzen. Die optimalen Kombinationen von Polymersubstrat, Initiator und Monomer für die Vorbehandlung lassen sich durch orientierende Versuche unschwer ermitteln. Beispielsweise sind (Meth)acrylsäure und/oder ihre Ester in Verbindung mit den vorerwähnten bevorzugten Photoinitiatoren gut für die Vorbehandlung von Polyamid-, Polyurethan-, Polyetherblockamid- oder Polyesteramid- oder -imid-Substraten geeignet.

Es ist zweckmäßig, daß die Mischung zur Vorbehandlung des Polymersubstrats zumindest überwiegend aus dem Initiator und mindestens einem Monomer besteht. Die Mischung kann also ausschließlich aus den genannten Bestandteilen bestehen oder allenfalls untergeordnete Menge, z.B. bis zu 40, vorteilhaft bis zu 20 Gewichtsprozent eines Lösemittels enthalten. Letzteres kann z.B. dann der Fall sein, wenn sich das Monomer und der Initiator nicht oder nicht gut zu einer homogenen Mischung oder Lösung mischen lassen oder das Substrat mit dem Monomer allein zu stark quillt. Geeignete Lösemittel sind z.B. die zuvor als Lösemittel für den Initiator genannten. Die kürzeren Bestrahlungszeiten des erfindungsgemäßen Verfahrens gehen wesentlich darauf zurück, daß Lösemittel in der Mischung zur Vorbehandlung des Polymersubstrats allenfalls in untergeordneten Mengen zugegen sind.

Die Behandlung des Polymersubstrats mit dem Initiator und dem Monomer soll so erfolgen, daß die Oberfläche des Polymersubstrats leicht quillt. Die Behandlungsdauer hängt von der jeweiligen Kombination von Polymersubstrat, Initiator und Monomer sowie von der Temperatur ab. Sie braucht nur 1 bis 10 Sekunden zu betragen und beträgt vorteilhaft 1 bis 5 Sekunden. Die optimalen Temperaturen und Behandlungszeiten lassen sich unschwer durch orientierende Versuche ermitteln. Vorzugsweise erfolgt die Behandlung des Polymersubstrats mit dem Initiator und dem aliphatisch ungesättigten Monomer bei einer Temperatur von 10 bis 200°C, besonders bevorzugt bei Temperaturen von 20 bis 80°C und insbesondere bei 30 bis 60°C.

Die den Initiator enthaltende Mischung zur Behandlung des Polymersubstrats kann durch übliche Auftragetechniken, wie Aufsprühen, Bestreichen oder Tauchen, auf das Polymersubstrat aufgebracht werden.

Auf das mit einem Initiator versehene Polymersubstrat werden erfindungsgemäß photochemisch oder thermisch induziert die Monomeren A, B und gegebenenfalls D oder das Polymer C und ein Vernetzer gepfropft. Die Monomeren bzw. das Polymer C können bei der Pfropfung in Substanz oder zweckmäßig in einem Lösemittel gelöst in Kontakt mit dem Polymersubstrat kommen. Als Lösemittel eignen sich, je nach der Art des Monomers bzw. des Polymers C, diejenigen, die auch für die Vorbehandlung geeignet sind. Man arbeitet im allgemeinen mit Lösungen, die 2 bis 50 Gewichtsprozent Monomere bzw. Polymer C enthalten. Das Polymersubstrat kann getaucht oder nach einem der erwähnten Auftragverfahren mit einer Lösung der Monomeren bzw. des Polymers beschichtet werden.

Die photochemische Pfropfung kann durch elektromagnetische Strahlung im Wellenlängenbereich von 180 bis 1.200 nm, bevorzugt von 200 bis 800 nm und insbesondere von 200 bis 400 nm induziert werden. Strahlung in diesem Wellenlängenbereich ist relativ weich und greift in aller Regel das Polymersubstrat nicht an. Man arbeitet z.B. mit einem Excimer-UV-Strahler der Fa. Heraeus. D-63801 Neuostheim mit kontinuierlicher Strahlung, z.B. mit XeCl oder XeF als Strahlermedium. Im Prinzip sind auch Quecksilberdampflampen mit breitem UV-Spektrum und Strahlungsanteilen im sichtbaren Bereich bzw. in den obengenannten Bereichen brauchbar. Die Expositionszeiten betragen im allgemeinen 60 bis 300 Sekunden. Die Expositionszeiten hängen u.a. von der Geometrie der bestrahlten Substrate ab. Gegenstände mit ausgeprägter dreidimensionaler Charakteristik müssen gedreht werden und erfordern längere Bestrahlung.

Alternativ kann die Pfropfung auch thermisch induziert werden. Man erhitzt dazu das mit den Monomeren A, B und gegebenenfalls C bzw. mit dem Polymer C und einem Monomer B beschichtete Polymersubstrat auf eine Temperatur, bei der der eingesetzte Initiator unter Radikalbildung zerfällt und die Pfropfung abläuft. Das ist je nach Initiator und Monomeren A, B und gegebenenfalls D bzw. Polymer C und Monomer B im allgemeinen der Fall, wenn man das Polymersubstrat 5 bis 240 Minuten auf eine Temperatur im Bereich von 50 bis 100°C erhitzt.

Nach der Pfropfung (gemäß 6.1 und 6.2) können eingeführte Gruppen in üblicher Weise ganz oder teilweise in Derivate überführt werden. So kann man Carboxylgruppen zu Carboxylatgruppen oder Sulfosäuregruppen zu Sulfonatgruppen neutralisieren, Carbonestergruppen zu Hydroxyl- oder Carbonsäuregruppen sowie Carbonamid- oder Nitrilgruppen zu Carboxylgruppen verseifen. Weitere Derivatisierungen von erfindungsgemäß modifizierten Polymersubstraten können nach allgemeinen Verfahren (H. Beyer, Lehrbuch der organischen Chemie, S. Hirzel Verlag. Stuttgart, 1988, S. 260 ff) vorgenommen werden.

Wenn es, insbesondere bei medizintechnischen Verwendungen, darauf ankommt, daß die gepfropften Beschichtungen frei von löslichen Monomeren oder Oligomeren sind, kann man diese unerwünschten Komponenten extrahieren, z.B. mit heißem Wasser. Da die Beschichtung kovalent auf dem Polymersubstrat gebunden ist, wird sie dadurch nicht abgelöst, beschädigt oder in ihrer biologischen Wirksamkeit beeinträchtigt.

### 7. Verwendung der beschichteten Substrate

Die erfindungsgemäß hergestellten beschichteten Polymersubstrate eignen sich für die Herstellung von Erzeugnissen oder sind Erzeugnisse zur Verwendung insbesondere auf den Gebieten der Nahrungs- und Genußmitteltechnik, Wassertechnik, Biotechnik, der Hygienevorsorge und insbesondere der Medizintechnik bestimmt. Solche Erzeugnisse, die ganz oder teilweise erfindungsgemäß beschichtet sein können, sind ebenfalls Gegenstand der Erfindung. Beispiele hierfür Textilien, Möbel und Geräte, Rohre und Schläuche, Fußböden, Wand- und Deckenflächen, Lagerbehälter, Verpackungen, Fensterrahmen, Telefonhörer, Toilettensitze, Türgriffe, Griffe und Haltegurte in öffentlichen Verkehrsmitteln und medizintechnische Artikel. Medizintechnische Artikel sind beispielsweise Implantate oder Hilfsmittel, wie Drainagen, Führungsdrähte, Kanülen, Intraokularlinsen, Kontaktlinsen, Stents, Gefäßprothesen, Gelenkprothesen, Knochenersatzmaterialien, Bandprothesen, Zahnprothesen für die plastische Chirurgie, Blutbeutel, Dialysemembranen, Nahtmaterialien, Verbandstoffen, Vliesprodukte und Operationsbestecke. Eine bevorzugte Verwendung der Materialien nach der Erfindung ist die zur Herstellung von Kathetern.

Die folgenden Beispiele sollen die Erfindung weiter erläutern, nicht jedoch ihren Anwendungsbereich begrenzen, wie er in den Patentsprüchen definiert ist.

### Beispiele 1 bis 6

In den Beispielen 1 und 2 wurden Polymere, in den Beispielen 3 bis 6 wurden Monomere gepfropft. Die

### Lösungen für die Beschichtungsversuche

wurden wie folgt hergestellt:

### Beispiel 1

1-molare Lösungen von Natrium-4-styrolsulfonat (NaSS). Maleinsäure (MS) und Polyethylenglykol-1000-monomethylethermonoacrylat (PEG-1000-MeO-MA; Fa. Polyscience Europe GmbH. Heidelberg) wurden im Volumenverhältnis von 5:5:1 vereinigt und mit 1 Mol-%, bezogen auf die Summe der Monomeren, K₂S₂O₈ versetzt. Der Ansatz wurde 4 Stunden unter Rühren auf 60°C erhitzt, abgekühlt und langsam und unter Rühren in das 5-fache Volumen Ethanol gegossen. Das ausgefällte Copolymer wurde abfiltriert, mit Ethanol gewaschen und 10 Stunden bei 55 °C im Umlufttrockenschrank getrocknet. Für die Beschichtungsversuche wurde eine 10 gewichtsprozentige wässerige Lösung des Copolymers hergestellt. Diese Lösung wurde mit soviel Diethylenglykoldimethacrylat (DEGDMA; Fa. Polyscience Europe GmbH, Heidelberg) als Vernetzer versetzt, daß dessen Konzentration 20 Gew.-% betrug.

### Beispiel 2

Es wurde wie im Beispiel 1 verfahren. Das Volumenverhältnis der Lösungen betrug jedoch 50:50:1, die hergestellte Lösung war 1 gewichtsprozentig, und der Vernetzer war Polyethylenglykol-1000-dimethacrylat (PEG1000DMA; Fa. Polyscience Europe GmbH, Heidelberg) in einer Konzentration von wiederum 20 Gew.-%.

### Beispiele 3 bis 6

Die Monomeren NaSS und Maleinsäure (MS) bzw. Acrylsäure (AS) wurden in den aus der Tabelle ersichtlichen Konzentrationen in Wasser gelöst. Die Art und die Konzentration der Vernetzer ist ebenfalls aus der Tabelle ersichtlich (PETAE = Pentaerythrittriallylether, PETA = Pentarythrittetraacrylat; Fa. Polyscience Europe GmbH, Heidelberg)

### Polymersubstrate und deren Aktivierung

Als Substratmaterial diente in allen Fällen eine 0,1 mm starke Polyamid 12-Folie (Typ L2101 der Hüls AG). Die Folie wurde vor der Beschichtung wie folgt aktiviert:

### Beispiel 1

Die Polyamid-Folie wurde 5 Sekunden in eine auf 70°C erwärmte Lösung von 40 g 2,2'-Dimethoxy-2-phenylacetophenon ("UV-Initiator") getaucht und an der Luft getrocknet.

### Beispiele 2 bis 6

Die Polyamid-Folien wurden jeweils 5 Minuten bei einem Druck von 1 mbar einer Excimerstrahlung von 172 nm Wellenlänge ausgesetzt. Die Strahlung stammte von einem Excimer-UV-Strahler (Heraeus, Kleinostheim, BR Deutschland) mit einer Leistung von 1,7 kW, Abstand zur Probe 4 cm.

### Pfropfpolymerisation

### Beispiele 1 bis 6

Die aktivierten PA 12-Folien wurden in die Beschichtungslösungen getaucht und im getauchten Zustand für die in der Tabelle angegebenen Zeiten einer Excimer-UV-Strahlung einer Wellenlänge von 308 nm ausgesetzt. Die Strahlung stammte wiederum von einem Excimer-UV-Strahler (Heraeus, Kleinostheim, BR Deutschland) mit einer Leistung von 1,7 kW, Abstand zur Probe 4 cm.

### Messung der Bakterienadhäsion beschichteter Standardfolien über ATP (statisch)

Es wird eine in der Technik übliche bioluminometrische Methode angewandt. Nach Adsorption von Bakterienzellen des Stammes Klebsiella pneumoniae an erfindungsgemäß behandelten bzw. unbehandelten PA 12-Folien werden die nichtadhärierten Bakterien mit steriler PBS-Pufferlösung weggespült. Aus den an der Folie adhärierenden Bakterien wird der Bakterieninhaltsstoff Adenosintriphosphat (ATP) extrahiert und mit einer handelsüblichen Testkombination im bioluminometrischen Test bestimmt. Die Anzahl der gemessenen Lichtimpulse ist proportional der Zahl an adhärierenden Bakterien.

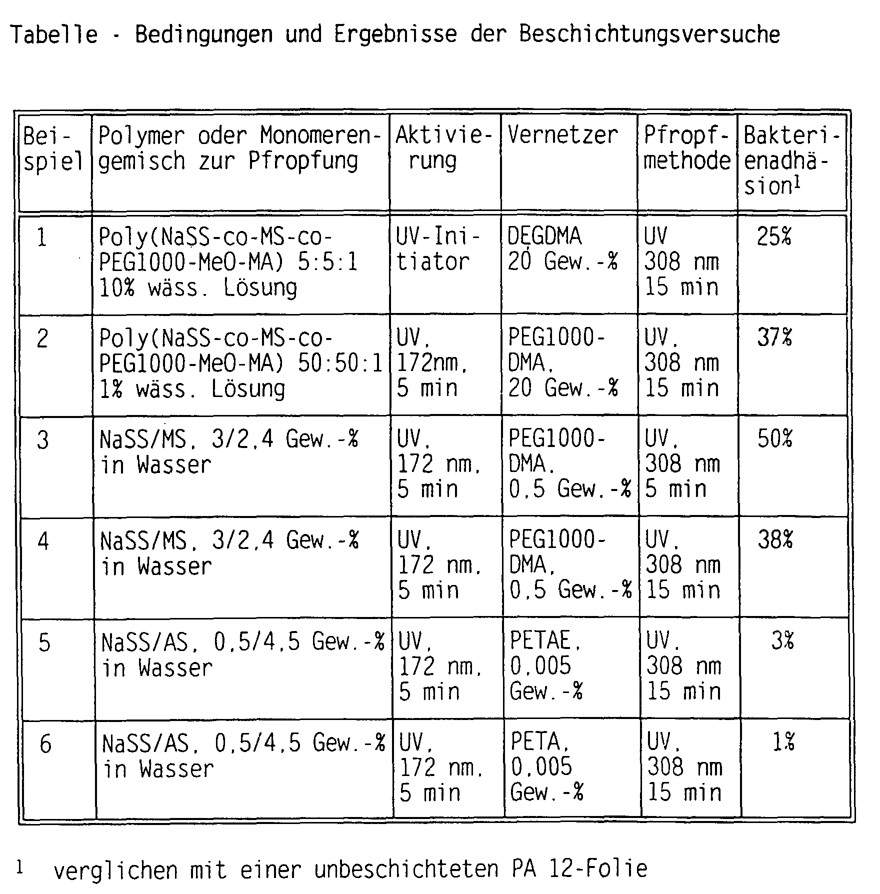

## Patentansprüche

1. Verfahren zur Beschichtung von Polymersubstraten, dadurch gekennzeichnet, daß man
(A) mindestens ein Vinylmonomer (Monomer A) der allgemeinen Formel 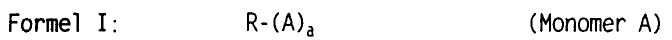 in der
R einen ein oder zweifach aliphatisch ungesättigten organischen Rest mit der Wertigkeit a bedeutet,
A eine Carboxylgruppe -COOH, Schwefelsäuregruppe -OSO₂OH, Sulfonsäuregruppe -SO₃H, Phosphorsäuregruppe -OPO(OH)₂, Phosphonsäuregruppe -PO(OH)₂, Phosphorigsäuregruppe -OP(OH)₂, phenolische Hydroxylgruppe oder ein Salz einer der genannten Gruppen bezeichnet, und
a für 1, 2 oder 3 steht;
mit der Maßgabe, daß wenn das Monomer der Formel I eine Carboxylgruppe -COOH oder einer Carboxylatgruppe aufweist, entweder dieses Monomer mindestens einen weiteren Rest A mit einer anderen der für A genannten Bedeutungen enthält oder mindestens ein weiteres Monomer der Formel I, in dem A eine andere der für A genannten Bedeutungen hat, mitverwendet wird; und
(B) mindestens ein mindestens zwei olefinische Doppelbindungen enthaltendes, vernetzendes Vinylmonomer (Monomer B) oder
(C) ein Polymer, das mindestens ein Monomer A enthält (Polymer C), mit mindestens einem Monomer B auf das Polymersubstrat pfropft; wobei entweder das Polymersubstrat vor der Pfropfung aktiviert und die Pfropfung durch elektromagnetische Strahlung oder thermisch initiiert wird oder
ein Initiator mitverwendet und die Pfropfung durch elektromagnetische Strahlung oder thermisch initiiert wird.

2. Verfahren nach Anspruch 1, dadurch gekennzeichnet, daß man neben den Monomeren A und B weitere, einfach olefinisch ungesättigte Monomere D mitverwendet, die hinsichtlich ihrer biologischen Wirkung neutral oder allenfalls schwach wirksam sind.

3. Verfahren nach einem der Ansprüche 1 oder 2, dadurch gekennzeichnet, daß man als Monomer A eine Mischung von Monomeren der allgemeinen Formeln II oder III 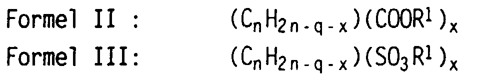 einsetzt, in denen
n jeweils unabhängig für eine ganze Zahl von 2 bis einschließlich 6;
x jeweils unabhängig für 1 oder 2;
q jeweils unabhängig für 0 oder 2 steht; und
R¹ jeweils unabhängig -H, ein Äquivalent eines Metallions,
einen Rest eines aliphatischen, cycloaliphatischen oder araliphatischen Alkohols bedeutet.

4. Verfahren nach einem der Ansprüche 1 oder 2, dadurch gekennzeichnet, daß man als Monomer A eine Benzolverbindung der Formel IV 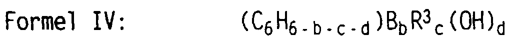 einsetzt, in der
B jeweils unabhängig einen ein- oder zweifach ungesättigten geradkettigen oder verzweigten Rest der Formeln (CₙH_{2n-1-q-y})(COOR¹)_{y} oder (CₙH_{2n-1-q-y})(SO₃R¹)_{y} bedeutet, wobei R¹, n und q wie in Anspruch 3 definiert sind und y für 0, 1 oder 2 steht;
R³ jeweils unabhängig C₁₋₄-Alkyl, -NH₂, -COOH, -SO₃H, -OSO₃H, -OPO(OH)₂, -PO(OH)₂, -OP(OH)₂, -OPO(O⁻)OCH₂-CH₂-N⁺(CH₃)₃, -PO(O⁻)O-CH₂-CH₂-N⁺(CH₃)₃, -OP(O⁻)OCH₂-CH₂-N⁺(CH₃)₃, ein Salz oder einen Ester der genannten Gruppen bedeutet;
b für 1, 2 oder 3 steht;
c für 0, 1, 2 oder 3 steht; und
d für 0, 1, 2 oder 3 steht;
mit der Maßgabe, daß b + c + d ≤ 6, vorteilhaft ≤4 ist.

5. Verfahren nach einem der Ansprüche 1 oder 2, dadurch gekennzeichnet, daß man als Monomer A eine Mischung aus Monomeren der allgemeinen Formeln V und VI 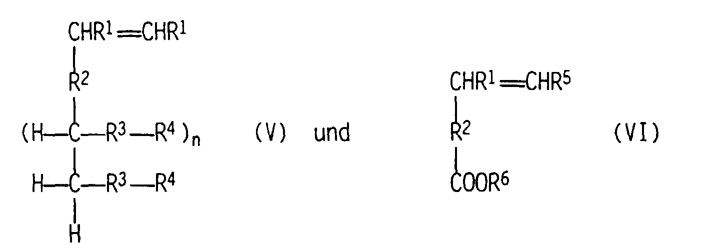 verwendet, in denen
R¹ Wasserstoff oder den Methylrest,
R² einen zweiwertigen organischen Rest oder eine C-C-Einfachbindung,
R³ -O- oder -NH-,
R⁴ Wasserstoff oder den Rest -SO₃⁻Na⁺
R⁵ Wasserstoff, den Methylrest oder den Rest -R²-COOR⁶,
R⁶ Wasserstoff oder Na und
n 4 oder 5 bedeuten;
mit der Maßgabe, daß mindestens einer der Substituenten R⁴ ein Rest -SO₃⁻Na⁺ ist.

6. Verfahren nach Anspruch 3, dadurch gekennzeichnet, daß die Monomeren A der Formeln II und III so gewählt werden, daß die gepfropfte Polymerschicht Carboxyl- und Sulfonsäuregruppen, Carboxyl- und Sulfonatgruppen, Carboxylat- und Sulfonatgruppen, Carboxyl-, Carboxylat- und Sulfonatgruppen oder Carboxyl-, Sulfosäure- und Sulfonatgruppen enthält.

7. Verfahren nach Anspruch 6, dadurch gekennzeichnet, daß das molare Verhältnis von Carboxyl- und/oder Carboxylatgruppen zu Sulfonsäure- und/oder Sulfonatgruppen 0,2 bis 3 beträgt.

8. Verfahren nach Anspruch 6, dadurch gekennzeichnet, daß das molare Verhältnis von Carboxyl- und/oder Carboxylatgruppen zu Sulfonsäure- und/oder Sulfonatgruppen 2 bis 10 beträgt.

9. Verfahren nach Anspruch 6, dadurch gekennzeichnet, daß das molare Verhältnis von Carboxyl- und/oder Carboxylatgruppen zu Sulfonsäure- und/oder Sulfonatgruppen 3 bis 5 beträgt.

10. Verfahren nach einem der Ansprüche 1 bis 9, dadurch gekennzeichnet, daß das vernetzende Vinylmonomer (Monomer B) 3 bis 6 olefinische Doppelbindungen aufweist.

11. Verfahren nach Anspruch 10, dadurch gekennzeichnet, daß das Monomer B keine Gruppen enthält, die zu der biologischen Wirkung der Beschichtung beitragen.

12. Verfahren nach einem der Ansprüche 1 bis 11, dadurch gekennzeichnet, daß man ein Polymer C aus mindestens einem Monomer A und gegebenenfalls mindestens einem Monomer D herstellt und dieses zusammen mit mindestens einem Monomer B auf das Polymersubstrat pfropft.

13. Verfahren nach einem der Ansprüche 1 bis 12, dadurch gekennzeichnet, daß die Monomeren A, B und gegebenenfalls D oder das Polymer C und das Monomer B auf aktivierte Oberflächen des Polymersubstrats thermisch oder durch Strahlung induziert gepfropft werden und die Aktivierung der Substratoberflächen durch UV-Strahlung, Plasmabehandlung, Coronabe- Behandlung mit einer starken Säure oder starken Base erfolgt.

14. Verfahren nach Anspruch 13, dadurch gekennzeichnet, daß man die Pfropfung durch Strahlen im Bereich von 250 bis 500 nm bewirkt.

15. Verfahren nach einem der Ansprüche 1 bis 12, dadurch gekennzeichnet, daß die Monomeren A, B und gegebenenfalls D oder das Polymer C und das Monomer B mittels Initiatoren gepfropft werden, welche unter dem Einfluß von elektromagnetischer Strahlung oder von Wärme unter Radikalbindung zerfallen und mit denen das Polymersubstrat vor der Pfropfung behandelt wurde.

16. Verfahren nach Anspruch 15, dadurch gekennzeichnet, daß die Behandlung erfolgt, indem man auf das das Polymersubstrat den Initiator zusammen mit einem Monomer einwirken läßt.

17. Verwendung von Polymersubstraten, die gemäß den Ansprüchen 1 bis 16 beschichtetet wurden, für die Herstellung von Erzeugnissen zur Verwendung auf den Gebieten der Nahrungs- und Genußmitteltechnik, Wassertechnik, Biotechnik, der Hygienevorsorge und insbesondere der Medizintechnik.

18. Erzeugnisse, die ganz oder teilweise gemäß den Ansprüchen 1 bis 16 beschichtet wurden, zur Verwendung auf den Gebieten der Nahrungs- und Genußmitteltechnik, Wassertechnik, Biotechnik, der Hygienevorsorge und insbesondere der Medizintechnik.

19. Erzeugnisse nach Anspruch 18, dadurch gekennzeichnet, daß die Erzeugnisse Textilien, Möbel und Geräte, Rohre und Schläuche, Fußböden, Wand- und Deckenflächen, Lagerbehälter, Verpackungen, Fensterrahmen, Telefonhörer, Toilettensitze, Türgriffe, Griffe und Haltegurte in öffentlichen Verkehrsmitteln und medizintechnische Artikel sind.

20. Erzeugnisse nach Anspruch 19, dadurch gekennzeichnet, daß die medizintechnischen Artikel Drainagen, Führungsdrähte, Kanülen, Intraokularlinsen, Kontaktlinsen, Stents, Gefäßprothesen, Gelenkprothesen, Knochenersatzmaterialien, Bandprothesen, Zahnprothesen für die plastische Chirurgie, Blutbeutel, Dialysemembranen, Nahtmaterialien, Verbandstoffen, Vliesprodukte, Operationsbestecke oder Katheter sind.
